# EUROPEAN PATENT APPLICATION

(11) **EP 3 457 132 A1**
(43) Date of publication of application: **20.03.2019**
(21) Application number: 17382606.6
(22) Date of filing: 13.09.2017
(51) Int. Cl.: G01N 33/50

(54) **METHOD FOR SCREENING COMPOUNDS THAT MODULATE THE ACTIVITY OF THE ELECTRON TRANSPORT CHAIN**

(71) Applicant: IMG Pharma Biotech, S.L., 48160 Derio-Bizkaia (ES)
(72) Inventor: BARREDA GÓMEZ, Gabriel, E-48160 Derio-Bizkaia (ES); ASTIGARRAGA ARRIBAS, Egoitz, E-48160 Derio-Bizkaia (ES)
(74) Representative: ABG Intellectual Property Law, S.L.

(57) **Abstract**

The invention relates to a method for determining the activity of a component of the electron transport chain or any associated protein thereof and for determining the effect of a compound on the activity of a component of the electron transport chain or any associated protein thereof The invention also relates to a kit comprising a redox probe, at least one electron donor compound of a component of the electron transport chain or a protein associated thereof and optionally comprising a sample comprising mitochondrial membranes and to the use of said kit in the methods of the invention.

## Description

### Technical Field of Invention

The present invention relates to a method for determining the activity of a component of the electron transport chain, such as individual complexes or supercomplexes and for determining the effect of a compound on said activity.

### Background of Invention

Oxidative phosphorylation (OXPHOS) is performed in the mitochondrial membrane by five complexes (CI, CII, CIII, CIV and CV) together with the mobile electron carriers, coenzyme Q (CoQ) and cytochrome c (cyt c). CI is composed of 45 subunits in humans, being transmembrane mitochondrial complex the largest. The main function of CI is to oxidize NADH and the electrons generated by this complex, as well as those produced by CII, are transferred through the electron transport chain (ETC) to O₂, generating a proton gradient. The functional assays, used for studying the ETC and for the screening of compounds that modify this mitochondrial activity, require whole mitochondrias isolated from fresh tissue, limiting their use in drug discovery.

Although initial studies shown that ETC components can coexist in a supramolecular structure named oxysome, further experimental evidences indicated that they can diffuse as individual units according to a fluid model in which electrons are transported by a diffusion-coupled process. However, in recent years, several studies demonstrated that CI, CIII and CIV can be associated to form different supramolecular structures known as supercomplexes (SC). The most abundant is the respirasome, which consists of CI, a CIII dimer and CIV. These structures seem to enhance the efficiency of OXPHOS, reducing the production of reactive oxygen species by creating CoQ and cyt c specific pools.

However, the lack of structural barriers that support the substrate channelling, together with the free diffusion of electron carriers inside and outside the SC structure observed, raises doubts about the functional relevance of these mitochondrial SC.

Therefore, there is a need in the art for methods for determining the activity of components of the electron transport chain and the identification of compounds capable of modulating said activity.

### Summary of the Invention

In a first aspect, the invention relates to a method for determining the activity of a component of the electron transport chain or any associated protein thereof, which comprises:
(i) putting into contact a sample comprising membranes containing a component of the electron transport chain or any associated protein thereof with a redox probe and at least one electron donor compound of any of said component or protein in conditions allowing the interaction between said electro donor compound and said corresponding component of the electron transport chain or associated protein thereof present in said sample and between said redox probe and a oxidative phosphorylation component of the electron transport chain; and
(ii) quantifying the signal obtained due to the oxidation or reduction of said redox probe.

In a second aspect, the invention relates to method for determining the effect of a compound on the activity of a component of the electron transport chain or any associated protein thereof, comprising:
(i) putting into contact the compound with a sample comprising membranes containing a component of the electron transport chain or any associated protein thereof in the presence of a redox probe, and at least one electron donor compound of any of said component or protein, in conditions allowing the interaction between said compound and a component of the electron transport chain or any associated protein thereof present in said sample, between said electron donor compound and its corresponding component of the electron transport chain or any associated protein thereof and between said redox probe and a oxidative phosphorylation component of the electron transport chain; and
(ii) quantifying the signal obtained due to the oxidation or reduction of said redox probe, wherein if the activity of a component of the electron transport chain or any associated protein thereof is increased, the compound is an inductor of one of the component of the electron transport chain or any associated protein thereof, and if the activity of a component of the electron transport chain or any associated protein thereof is reduced, the compound is an inhibitor of a component of the electron transport chain or any associated protein thereof.

In a third aspect, the invention relates to a kit comprising a redox probe, at least one electron donor compound of a component of the electron transport chain or of a protein associated thereof and optionally a sample comprising mitochondrial membranes.

In a fourth aspect, the invention relates to the use of a kit of the invention in a method of the invention.

### Brief Description of the Figures

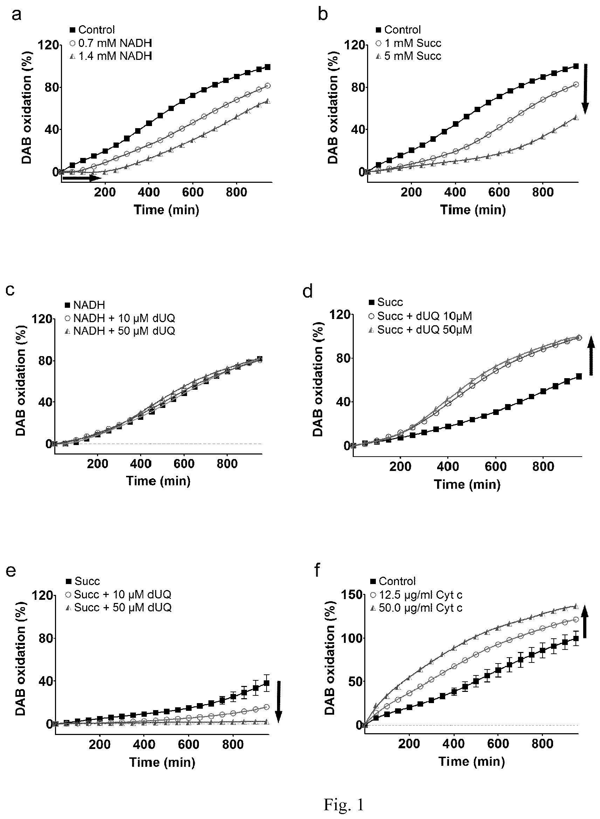

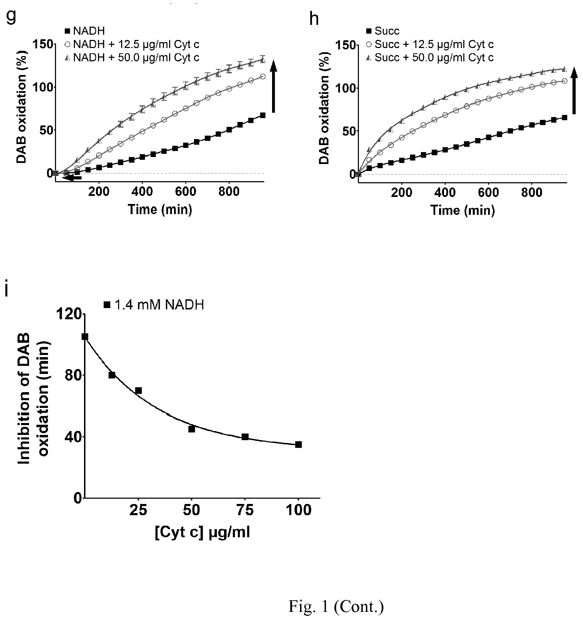

**Figure 1****.** Influence of electron carriers on DAB oxidation and implications on supercomplex function. (a,b) Concentration dependent inhibition of the DAB electron input induced by the CI substrate, NADH (a) or by the CII substrate, succinate (b). (c-e) Effects of dUQ on the inhibition induced by NADH (0.7 mM) (c) or by succinate at 1 mM (d) or at 5 mM (e). (f-i) Effects of soluble cyt c on DAB oxidation in absence (f) or in presence of NADH (g) or succinate (h). (i) Concentration dependent effects of cyt c on the inhibition time evoked by NADH.

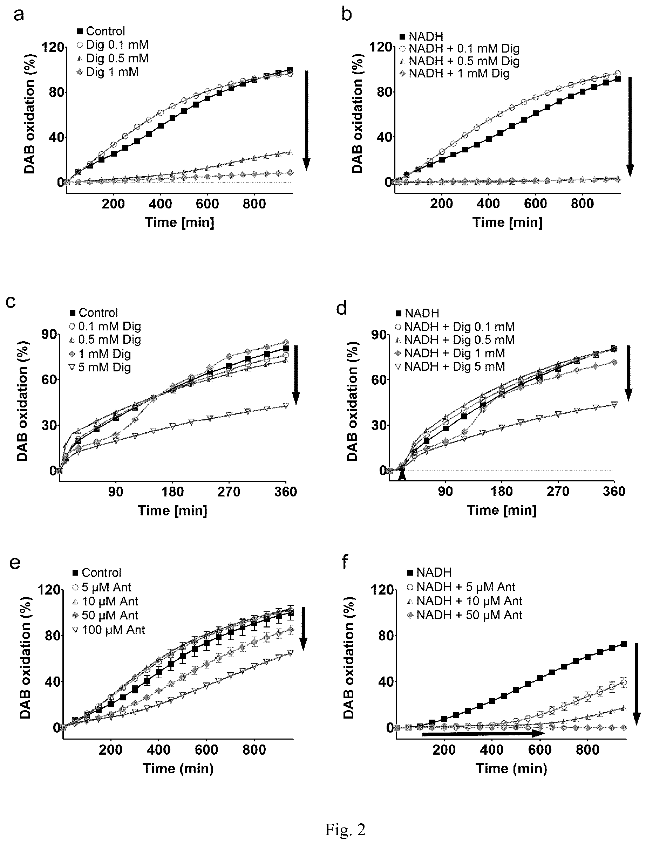

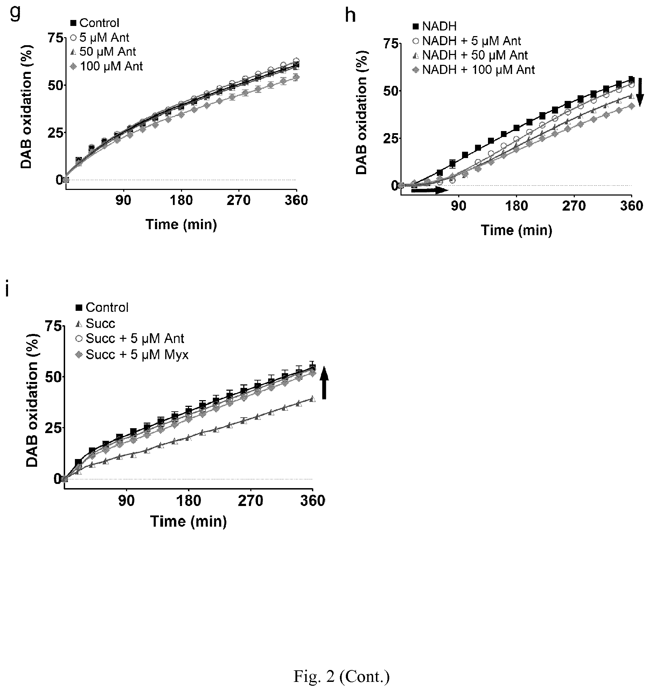

**Figure 2****.** Effects of digitonin and antimycin A on the endogenous cytochrome c cycle. (a-d) Effects of increasing concentrations of digitonin on the basal DAB oxidation (a,c) and on the supercomplex activity after stimulation with NADH (b,d) in absence (a,b) or in presence of cyt c (c,d). (e-h) Effects of increasing concentrations of antimycin A on the basal DAB oxidation (e,g) and on the supercomplex activity after stimulation with NADH (f,h) in absence (e,f) or in presence of cyt c (g,h). The presence of soluble cyt c in the assay medium reduces both effects indicating that digitonin and antimycin A induce the release of the endogenous cyt c. (i) Blockage of the succinate mediated electron input by CIII inhibitors, antimycin A and myxothiazol. These inhibitors induce opposite actions on DAB oxidation depending on the origin of the electron flux, according to the capacity of individual complex to form supercomplex structures.

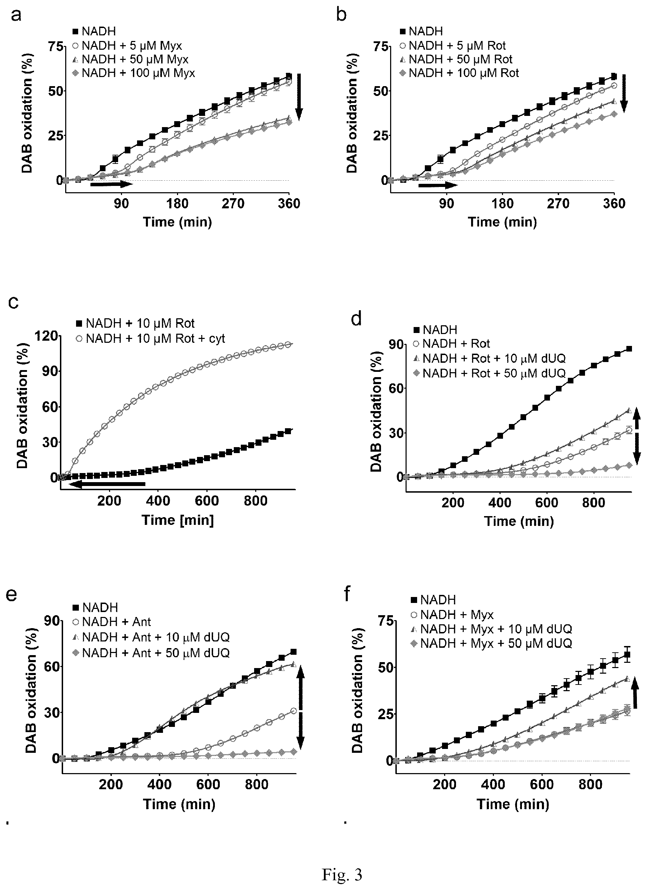

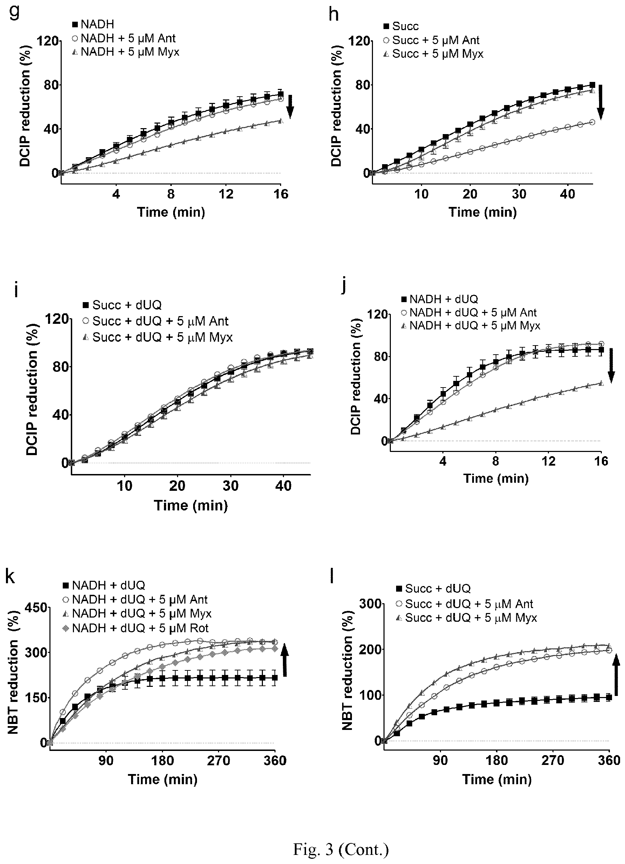

**Figure 3****.** Characterization of the supercomplex role in electron flux, ubiquinone reduction and superoxide production using specific inhibitors of the ETC. (a,b) Effects of myxothiazol (a) and rotenone (b) on the inhibition period of DAB oxidation induced by NADH in the presence of exogenous cyt c. The presence of ETC inhibitors together with the CI substrate increases the time period in which supercomplex is coupled, delaying, in this way, the electron input derived from DAB oxidation. (c) cyt c reduces the enhancement in the inhibition period evoked by rotenone. (d-f) Biphasic actions of dUQ on the blockage of DAB oxidation induced by NADH with rotenone (d), antimycin A (e) and myxothiazol (f). (g-j) Effects of CIII inhibitors on the ubiquinone reduction induced by NADH (0.7 mM) (g,j) or succinate (1 mM) (h,i) in absence (g,h) or in presence (i,j) of dUQ (50 µM). dUQ prevents the decrease in the production of ubiquinol by CII evoked by antimycin A (h,i), whilst it doesn't with that produced by CI in the presence of myxothiazol (g,j), according to a free diffusion model between CII and CIII or to a supercomplex model between CI and CIII. (k,l) Superoxide production evoked by ETC inhibitors using NADH (0.7 mM) (k) or succinate (1 mM) (1).

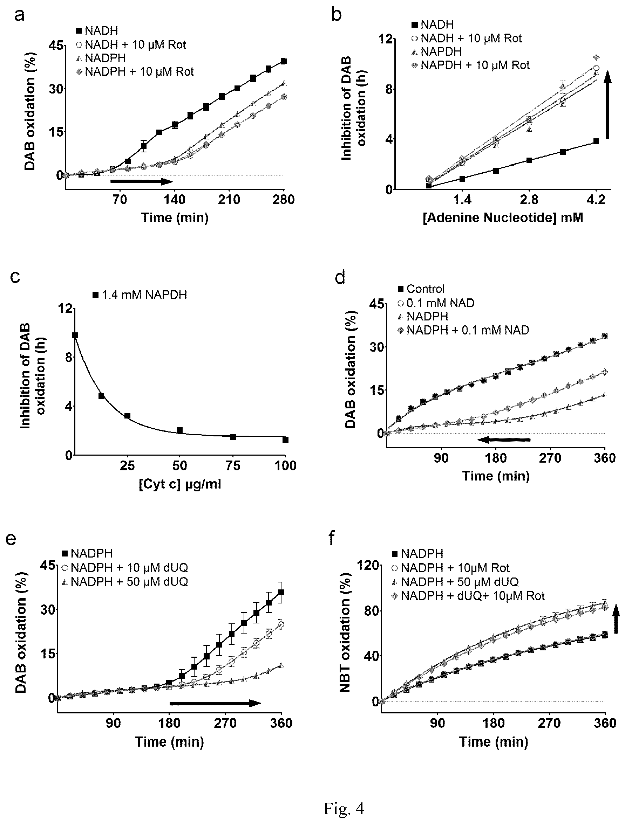

**Figure 4****.** Effects of NADPH on supercomplex activity. (a) NADPH (1.4 mM) induces a blockage of the DAB electron input similar to that produced by NADH (1.4 mM) in the presence of rotenone. (b) Relationship between the concentration of adenine nucleotides, with or without rotenone (10 µM), and the time period in which DAB oxidation is blocked. (c) Concentration dependent effects of cyt c on the inhibition time evoked by NADPH. (d) Effect of NAD (0.1 mM) on the blockage of DAB oxidation induced by NADPH. (e) dUQ potentiates the blockage of DAB oxidation induced by NADPH (2.8 mM). (f) Superoxide production evoked by NADPH (0.7 mM) in the absence and in the presence of rotenone (10 µM) and/or dUQ (50 µM).

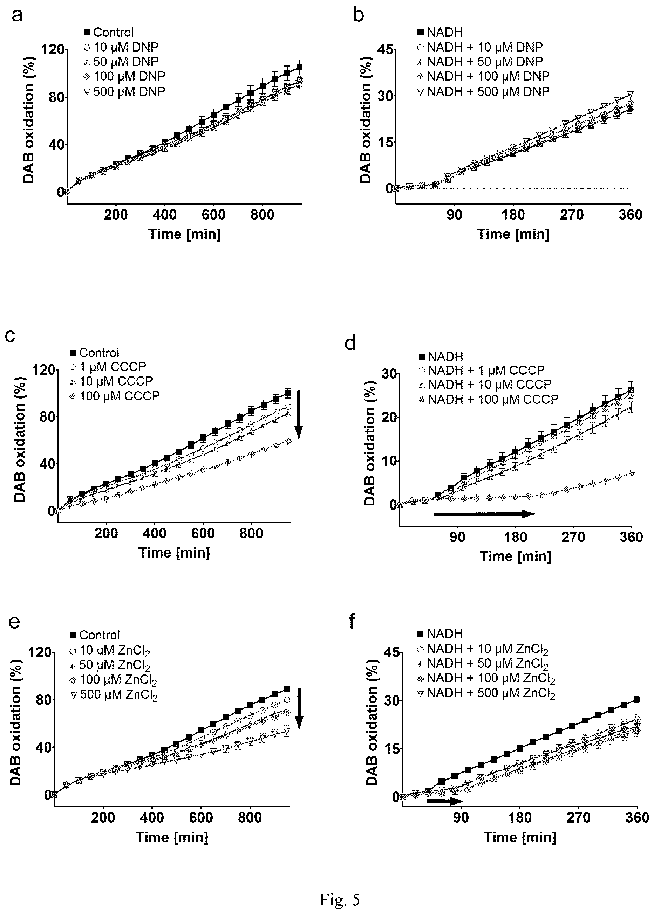

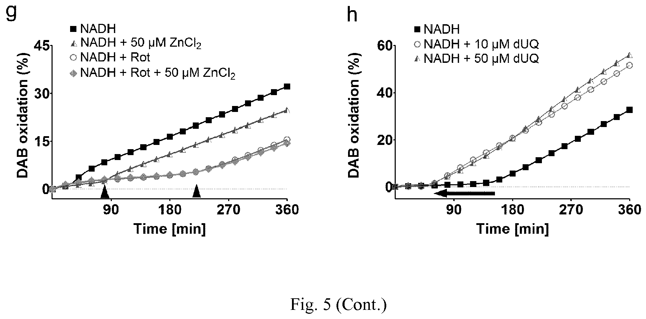

**Figure 5****.** Effects of protonophores and Zinc on electron flux and specific differences between rat and monkey. (a-d) Effects of increasing concentrations of the protonophores DNP (a,b) and CCCP (c,d) on the basal DAB oxidation (a,c) and on the supercomplex activity after stimulation with NADH (1.4 mM) (b,d). Influence of Zinc on the basal DAB oxidation (e) and on the supercomplex activity after stimulation with NADH (0.7 mM) in absence (f) or in presence of rotenone (10 µM) (g). (h,i) Effects of dUQ on the inhibition induced by NADH (0.7 mM) alone (h) or with rotenone (i) determined in MMP isolated from heart of cynomolgus monkey.

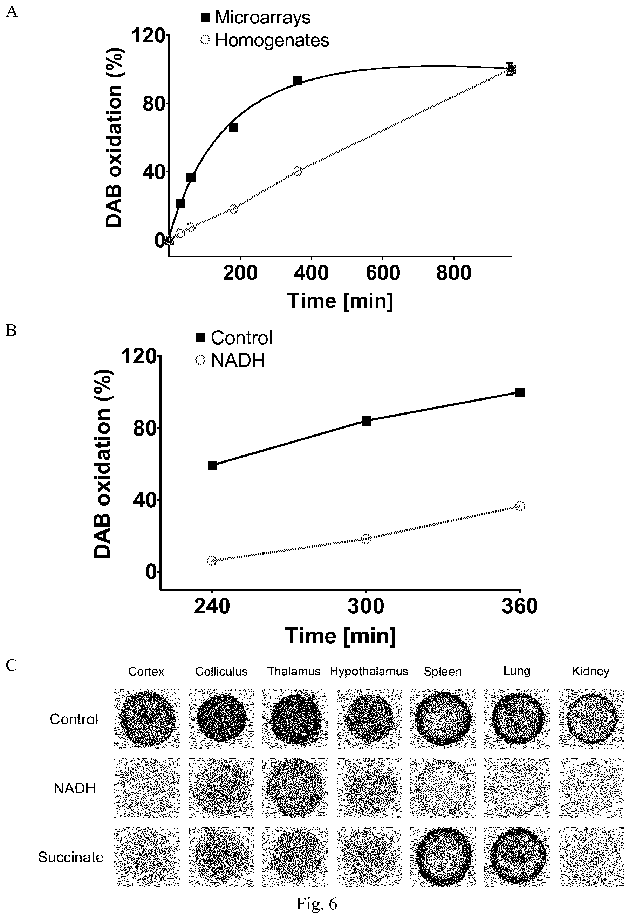

**Figure 6****.** Characterization of DAB protocol to detect supercomplex activity in cell membrane microarrays. (a) Time dependent DAB oxidation determined in heart MMP using membrane homogenates or cell membrane microarrays. (b) Inhibition of the DAB electron input evoked by the CI substrate, NADH (10 µM). (c) Representative images of the MMP microspots isolated from different tissues after being assayed with or without NADH or succinate using cell membrane microarrays.

### Detailed Description of the Invention

The present invention relates to a method for studying the function of a component of the electron transport chain, including individual components, supercomplex structures or any associated protein of the ETC, as well as for screening and analyzing compounds that modulate the activity of said components of the mitochondrial ETC. The invention is specifically based on the quantification of a signal generated by a redox probe such as 3,3'-diaminobenzidine (DAB) in presence of cells or membranes with concentrations of electron donors that generate an electron input such as the substrates of the different components involved in the oxidative phosphorylation (NADH, NADPH, FADH2 or succinate, among others) and with and without a drug candidate. The competition among the electron flow evoked by these electron donors or substrates and that produced by the redox probe enables the determination of the mitochondrial activity in absence or in presence of a drug candidate, allowing the characterization of the pharmacological profile of said drug candidate.

The invention allows determining the activity not only of individual components of the electron transport chain or any associated protein thereof but also the association of several of these components in supercomplex structures.

### Method for determining the activity of mitochondrial components of the ETC

In a first aspect, the invention relates to a method for determining the activity of a component of the electron transport chain or any associated protein thereof (first method of the invention), which comprises:
(i) putting into contact a sample comprising membranes containing a component of the electron transport chain or any associated protein thereof with a redox probe and at least one electron donor compound of any of said component or protein in conditions allowing the interaction between said electro donor compound and said corresponding component of the electron transport chain or associated protein thereof present in said sample and between said redox probe and a oxidative phosphorylation component of the electron transport chain; and
(ii) quantifying the signal obtained due to the oxidation or reduction of said redox probe.

In a first step, the method for determining the activity of a component of the electron transport chain or any associated protein thereof comprises (i) putting into contact a sample comprising membranes containing a component of the electron transport chain or any associated protein thereof with a redox probe and at least one electron donor compound of any of said component or protein in conditions allowing the interaction between said electro donor compound and said corresponding component of the electron transport chain or associated protein thereof present in said sample and between said redox probe and a oxidative phosphorylation component of the electron transport chain.

"Component of the electron transport chain", as used herein refers to an individual component or to a mitochondrial supercomplex of the electron transport chain. The main mitochondrial components that form an integral part of oxidative phosphorylation are the complex I (NADH:ubiquinone oxidoreductase); the complex II (succinate dehydrogenase); the complex III (cytochrome bc1 complex); the complex IV (cytochrome c oxidase); the complex V (ATP synthase); the ubiquinone (coenzyme Q) and the cytochrome c. In spite of the structures and organization of these structures vary according to the specie, the function is maintained.

"Complex I" refers to NADH:ubiquinone oxidoreductase, NADH-CoQ reductase, or NADH dehydrogenase; EC 1.6.5.3, that is the first and major entrance point of electrons to the respiratory chain. It transfers electrons from NADH molecules to a lipophilic quinone designated ubiquinone.

"Complex II", refers to succinate dehydrogenase or succinate-CoQ reductase; EC 1.3.5.1, and transmits electrons from succinate to ubiquinone and directly connects the citric acid cycle to the respiratory chain.

"Complex III" refers to cytochrome bc1 complex or CoQH2-cytochrome c reductase; EC 1.10.2.2, and catalyzes the reduction of cytochrome c by oxidation of coenzyme Q (CoQ) and the concomitant pumping of 4 protons from the mitochondrial matrix to the intermembrane space.

"Complex IV", as used herein, refers to cytochrome c oxidase; EC 1.9.3.1, that is the last enzyme in the respiratory electron transport chain of mitochondria located in the mitochondrial membrane. It receives an electron from each of four cytochrome c molecules, and transfers them to one oxygen molecule, converting molecular oxygen to two molecules of water.

"Complex V" refers to ATP synthase, EC 3.6.3.14, is an important enzyme that creates the energy storage molecule adenosine triphosphate (ATP) and it consists of two regions, the FO portion embedded within the membrane and the F1 portion of the ATP synthase is outside the membrane, but inside the matrix of the mitochondria. The F_{O} component acts as an ion channel that provides for a proton flux back into the mitochondrial matrix. This reflux releases free energy produced during the generation of the oxidized forms of the electron carriers (NAD+ and Q). The free energy is used to drive ATP synthesis, catalyzed by the F₁ component of the complex.

"Cytochrome c" is a mobile electron carrier that diffuses through the intermembrane space shuttling electrons from the c1 heme of complex III to CuA site of complex IV.

"Ubiquinone (coenzyme Q)", is an essential electron carrier in the mitochondrial respiratory chain and an important antioxidant. It catalyzes the reduction of cytochrome c by oxidation of coenzyme Q (CoQ) and the concomitant pumping of 4 protons from the mitochondrial matrix to the intermembrane space, CoQ is a mobile electron carrier because its isoprenoid tail makes it highly hydrophobic and lipophilic.

In a preferred embodiment, the first method of the invention relates to the determination of the activity of a mitochondrial supercomplex.

"Mitochondrial supercomplex", as used herein, relates to a supramolecular structure in which different components involved in the oxidative phosphorylation coexist, conferring specific structural and functional features different from that observed in individual complexes. The most abundant is the respirasome, which consists of CI, a CIII dimer and CIV (I₁III₂IV₁), but other supercomplexes have been also exist combining these complexes (I₁III₂; I₁IV₄; III₂IV₁; III₂IV₂; I₁III₂IV₂ among others)

"Associated proteins of a component of the electron transport chain (ETC)", as used herein, refers to other mitochondrial proteins that can control the ETC function by the allosteric modulation of any of the ETC component or by inducing an electron input to the mitochondrial ETC such as fatty acid β-oxidation enzymes, glycerol 3-phosphate dehydrogenase, dihydroorotate dehydrogenase or protein kinase A, among others.

"Activity of a component of the electron transport chain or any associated protein thereof", as used herein relates to the oxidation of substrates generating an electron flow along the mitochondrial transport chain that ends with the reduction of an oxygen molecule. In this process, protons are translocated from the mitochondrial matrix to the intermembrane space triggering a proton gradient that can be used by complex V to produce ATP. However, reactive oxygen species can be also formed in the course of this respiratory process. Supercomplex structures reduce the diffusion distance among the different components, increasing in this way the efficiency of the electron transport and thus reducing the production of reactive oxygen species.

"Sample comprising membranes", as used herein relates to any sample comprising intact mitochondria membranes capable of maintaining the activity of a component of the electron transport chain or any associated protein thereof or other mitochondrial components. "Capable of maintaining the activity of a component of the electron transport chain or any associated protein thereof or other mitochondrial components" it is meant that the membranes or mitochondria are capable of maintaining the oxidative phosphorylation to transport electrons to oxygen and to produce ATP. In a preferred embodiment, the sample comprises mitochondria.

Samples suitable in the present invention are by way of illustrative non limitative example a body fluid, a tissue sample, an organ culture or any other tissue or cell preparation from a subject or a biological source, such as, for example, blood, serum, plasma, urine and saliva or from tissues such as heart. The sample may be obtained from any eukaryotic organism, particularly from mammals, more particularly from rat, mouse or human.

In a preferred embodiment, samples suitable in the present invention are membrane or cell solutions, microarrays, tissue sections or isolated mitochondria. In a preferred embodiment, the sample comprises a tissue sample, whole eukaryotic or prokaryotic cells, organelles, cellular membranes, liposomes, isolated mitochondrial or isolated mitochondrial membranes. Mitochondria may be isolated from the samples using methods known to those of ordinary skill in the art. The key steps when isolating mitochondria from any tissue or cell are always the same: (i) rupturing of cells by mechanical and/or chemical means and (ii) differential centrifugation at low speed to remove debris and extremely large cellular organelles, followed by centrifugation at a higher speed to isolate mitochondria which are collected. Commercially available kits for isolating mitochondria include, but are not limited to, Mitochondrial Isolation Kit, Catalog No. MITOISO2 (Sigma Aldrich, Inc., St. Louis, Mo.); Mitochondrial Isolation Kit for Tissue, Catalog No. MS850 (MitoSciences, Inc., Eugene, Ore.); Mitochondrial Isolation Kit, Order No. 130-094-532 (Miltenyi Biotech, Inc., Auburn, Cal.); and Mitochondrial Isolation Kit for Tissue, Catalog No. 89801 (Thermo Fisher Scientific, Rockford, Ill.). Mitochondrial membrane homogenates can be obtained by homogenization and centrifugation processes known in the state of the art starting from any tissue, organ or cells.

In another preferred embodiment, the sample is a cell membrane microarray. In another preferred embodiment, the sample is a mitochondrial membrane preparation (MMP). In a more preferred embodiment, said MMP is isolated from heart.

According to the method of the invention, the sample is contacted with a redox probe and at least one electron donor compound of a component of the electron transport chain or any associated protein thereof.

"Electron donor compound of a component of the electron transport chain or any associated protein thereof" as used herein relate to a compound that donate electrons to at least one of the proteins that integrate or are associated to the ETC, such as substrates of the complex I (NAD, NADPH, among others), complex II (succinate, FAD, among others) or the fatty acid β-oxidation enzymes (palmitoyl-CoA, acyl-CoA, among others). In a preferred embodiment, an electron donor compound of a component of the electron transport chain or any associated protein thereof is a substrate of said component of the electron transport chain or any associated protein thereof.

"A substrate of a complex of the electron transport chain or any associated protein thereof" as used herein relates to a compound upon which a member of the electron transport chain acts, particularly complex I, complex II, complex III, complex IV or complex V, fatty acid β-oxidation enzymes, glycerol 3-phosphate dehydrogenase, dihydroorotate dehydrogenase or protein kinase A, as a way of illustrative non limitative example.

By way of illustrative non-limitatives examples a substrate of complex I is NADH, NADPH, a substrate of complex II is succinate and FADH2, substrates of complex III are quinol, ferri cytochrome c or duroquinol, substrates of complex IV are TMPD and ascorbate and a substrate of complex V is ADP, substrate of fatty acid β-oxidation enzymes are palmitoyl-CoA or any other acyl-CoA, a substrate of glycerol 3-phosphate dehydrogenase is glycerol 3-phosphate and a substrate of dihydroorotate dehydrogenase is dihydroorotate.

As the skilled person in the art can understand, the activity of a particular component of the electron transport chain or the activity of a particular protein associate thereof can be analyzed putting into contact the sample with the redox probe and the particular electron donor compound of the component of the electron transport chain or protein associated thereof whose activity is to be analyzed.

"Redox probe", as used herein relates to an organic or inorganic compound which donates or accepts electrons to any mitochondrial component involved in the oxidative phosphorylation. In the present invention, the change in the chemical properties of the redox probe allows the quantification of the method. The redox probe is capable of giving rise to a chromogenic, fluorogenic, radioactive and/or chemiluminescent signal which allows the detection, identification and, optionally, quantification of the amount of the oxidative phosphorylation. In a particular embodiment, said probe is selected from the group consisting of radioisotopes, enzymes, fluorophores or any molecule susceptible to being conjugated with another molecule or detected and/or quantified directly. In a preferred embodiment, the redox probe is suitable for accepting or donating electrons to cytochrome C.

A person skilled in the art knows the redox potential of a probe suitable for accepting or donating electrons to cytochrome C. As a way of illustrative non - limitative example, when the redox probe is DAB, the reduction potential for this electron donor is approximately - 0.9 V.

The standard reduction potential (pH=7, T=25° C) of cytochrome c has been determined as E°=0.235 V.

According to this, and in standard conditions (pH=7, T=25° C) the reduction reaction will occur spontaneously in presence of any electron donor with E° < 0.235 V, while the oxidation (inverse) reaction will take place in presence of one electron acceptor with E° >0.235 V. As an example, the standard reduction potentials at pH=7 and half reactions for molecules involved in membrane redox, according to Redox Reactions and Electron Transfer Across the Red Cell Membrane (E. C. Kennett, P. W. Kuchel, IUBMB Life, 55(7): 375-385, July 2003*)* are the following:

| Substrate | | Product | Reduction potential (V) |
|---|---|---|---|
| ½ O₂+2H⁺+2e⁻ | ↔ | H₂O | 0.815 V |
| p-benzoquinone + 2H⁺ +2 e⁻ | ↔ | hydroquinone | 0.699 V |
| α-tocopheroxyl · +e⁻ | ↔ | α-tocopherol | 0.500 V |
| Ferricyanide + e⁻ | ↔ | ferrocyanide | 0.360 V |
| O₂(g)+2H⁺+2e⁻ | ↔ | H₂O₂ | 0.295 V |
| AFR+H⁺+e⁻ | ↔ | AA | 0.282 V |
| cytochrome c (Fe³⁺)+e⁻ | ↔ | cytochrome c(Fe²⁺) | 0.235 V |
| DCIP (oxidised) + e⁻ | ↔ | DCIP (reduced) | 0.220 V |
| DHA + e⁻ | ↔ | AA | 0.800 V (pH=6.4) |
| Ubiquinone+2H⁺+2e⁻ | ↔ | H₂O+ubiquinol | 0.450 V |
| DHA + e⁻ | ↔ | AFR | -0.174 V |
| Pyruvate⁻+2H⁺+2e⁻ | ↔ | lactate⁻ | -0.185 V |
| ½ GSSG+ e⁻ | ↔ | NADH | -0.320 V |

| | | | |
|---|---|---|---|
| GSSG: glutathione disulfide, AA: ascorbic acid, DHA: dehidroascorbic acid, AFR: ascorbic free radical and DCIP: dichloroindophenol | | | |

As an example, the standard reduction potentials of the respiratory chain carriers according to Guide to Biochemistry (James C. Blackstock, Ed.Butterworth-Heinemann, 2014, pp.163*)* are the following:

| COMPLEX | CARRIER | E° (V) |
|---|---|---|
| | NAD⁺ | -0.320 |
| **I** | FMN | ? |
| | FeS_{N-1a} | -0.370 |
| | FeS_{N-1b} | -0.245 |
| | FeS_{N-2} | -0.020 |
| | FeS_{N-3} | -0.245 |
| | FeS_{N-4} | -0.245 |
| | FeS_{N-5} | -0.270 |
| **II** | FAD | -0.180 |
| | Cytochrome b₅₆₀ | -0.080 |
| | FeS_{S-1} | 0.000 |
| | FeS_{S-2} | -0.260 |
| | FeS_{S-3} | 0.065 |
| | Ubiquinone | 0.065 |
| **III** | Cytochrome b₅₆₂ | 0.030 |
| | Cytochrome b₅₆₆ | -0.030 |
| | FeS_{R} | 0.280 |
| | Cytochrome c₁ | 0.230 |
| | Cytochrome c | 0.230 |
| **IV** | Cytochrome a | 0.250 |
| | Cytochrome a₃ | 0.385 |
| | O₂ | 0.820 |

A suitable redox probe may be a fluorescence redox probe. Fluorescence-based techniques have become more popular due to their sensitivity and relative ease of detection. Dichlorodihydrofluorescein (DCFH2), dihydrorhodamine (DHR), dichlorodihydrofluorescein diacetate (DCFH-DA) and hydroethidine (HE), which are reduced derivatives of commonly used fluorophores, have been the most popular fluorescent probes. Oxidation of the reduced fluorophore results in structural changes, leading to increased fluorescence. Other examples are OxyBURST Green H2DCFDA succinimidyl ester, BODIPY 581/591C11 (Invitrogen), fluorescein and hydroxyphenyl fluorescein. In addition, the redox probe may be conjugated, such as bovine serum albumin coupled to a reduced fluorescein derivative (dihydro-2',4,5,6,7,7'-hexafluorofluorescein, H2HFF), crosslinked to 3 µm carboxylated silica particles using the heterobifunctional crosslinker cyanamide. Upon oxidation the H2HFF compound emits fluorescence at 488 nm excitation/520 nm emission.

Suitable chemiluminescence redox probes in the present invention are luminol, isoluminol, lucigenin-enhanced chemiluminescence, MCLA and coelenterazine.

In a preferred embodiment, the redox probe is an electron donor probe. An electron donor probe, as used herein relates to a compound that donates electrons to any mitochondrial component involved in the oxidative phosphorylation competing with the electrons generated by the oxidation of substrates of the components of the electron transport or any protein associated thereof by electron donors or by the reduced forms of the mitochondrial electron carriers.

In a more preferred embodiment the electron donor probe is a chromogenic redox probe. Chromogenic redox probes that can be used in the present invention are for example Nitro blue tetrazolium salt (NBT) or other tetrazolium salts, 3,3',5,5'-Tetramethylbenzidine (TMB), 3,3'-Diaminobenzidine (DAB), ,N,N',N'-Tetramethyl-p-phenylenediamine dihydrochloride (TMPD), ascorbic acid, 2,2'-azino-bis(3-ethylbenzothiazoline-6-sulphonic acid) (ABTS) o-phenylenediamine dihydrochloride (OPD), 4-aminoantipyrine, hyidroquinone (HQ), 3,3',5,5'-Tetramethylbenzidine (TMB), Amplex Red, 3-amino-9-ethylcarbazole (AEC), homovanillic acid, pyrogallol, p-aminophenol (PAP), N,N-diethyl-p-phenylenediamine sulfate (DPD) or 1,2,3-trihydroxybenzene (THB) or any derivative thereof.

In an even more preferred embodiment, the electron donor is selected from the group consisting of 3,3'-diaminobenzidine (DAB); N,N,N',N'-Tetramethyl-p-phenylenediamine dihydrochloride (TMPD), ascorbic acid and any derivative thereof. In a more preferred embodiment, the redox probe is 3,3'-diaminobenzidine (DAB).

3,3'-diaminobenzidine (DAB), as used herein relates to an organic compound is chemically and thermodynamically stable and when is oxidized give a dark-brown color.

A derivative or analogue of the particular electron donor as a way of illustrative non limitative example refers to water soluble salts of said compounds, such as 3,3'-Diaminobenzidine tetrahydrochloride or DAB tablets.

In another preferred embodiment, the redox probe is an electron acceptor probe. An electron acceptor probe, as used herein, relates to an oxidizing agent and is itself reduced during the process of redox reaction.

In a more preferred embodiment, the electron acceptor compound is selected from the group consisting of 2,6-dichloroindophenol (DCIP); 2,6-Dichlorophenolindophenol (DCPIP); tetrazolium salts such as nitroblue tetrazolium (NBT) and any derivative thereof.

In a preferred embodiment, substrates of at least 2 complexes are used in the present invention. Particularly a substrate of complex I and a substrate of complex II; a substrate of complex I and a substrate of complex III; a substrate of complex I and a substrate of complex IV; a substrate of complex I and a substrate of complex V; a substrate of complex II and a substrate of complex III; a substrate of complex II and a substrate of complex IV; a substrate of complex II and a substrate of complex V; a substrate of complex III and a substrate of complex IV; a substrate of complex III and a substrate of complex V; or a substrate of complex IV and a substrate of complex V.

In another preferred embodiment, substrates of at least 3 complexes are used, particularly a substrate of complex I, a substrate of complex II and a substrate of complex III; a substrate of complex I, a substrate of complex II and a substrate of complex IV; a substrate of complex I, a substrate of complex II and a substrate of complex V; a substrate of complex I, a substrate of complex III and a substrate of complex IV; a substrate of complex I, a substrate of complex III and a substrate of complex V; a substrate of complex I, a substrate of complex IV and a substrate of complex V; a substrate of complex II, a substrate of complex III and a substrate of complex IV; a substrate of complex II, a substrate of complex III and a substrate of complex V; a substrate of complex II, a substrate of complex IV and a substrate of complex V; or a substrate of complex III, a substrate of complex IV and a substrate of complex V.

In another preferred embodiment, substrates of at least 4 complexes are used, particularly a substrate of complex I, a substrate of complex II, a substrate of complex III and a substrate of complex IV; a substrate of complex I, a substrate of complex II, a substrate of complex III and a substrate of complex V; a substrate of complex I, a substrate of complex II, a substrate of complex IV and a substrate of complex V; a substrate of complex I, a substrate of complex III, a substrate of complex IV and a substrate of complex V; or a substrate of complex II, a substrate of complex III, a substrate of complex IV and a substrate of complex V.

In another preferred embodiment substrates of at least 5 complexes are used, particularly a substrate of complex I, substrate of complex II, substrate of complex III, substrate of complex IV and a substrate of complex V.

In another preferred embodiment substrates of at least 5 complexes and of the associated enzymes of the ETC are used, particularly a substrate of complex I, substrate of complex II, substrate of complex III, substrate of complex IV, a substrate of complex V, a substrate of fatty acid β-oxidation enzymes, a substrate of glycerol 3-phosphate dehydrogenase and a substrate of dihydroorotate dehydrogenase.

In a more preferred embodiment, the method comprises a substrate of complex I and a substrate of complex II. In an even more preferred embodiment, the substrate of complex I is NADH or NADPH and the substrate of complex II is succinate or FADH2.

As the person skilled in the art understands, the amount of the substrates may not be a limiting factor.

Substrates of complexes of the electron transport chain are contacted with the compound, the sample comprising mitochondrial membranes and the redox probe in conditions allowing the interaction between said substrates and their corresponding complexes of the electron transport chain present in the sample and between said redox probe and an oxidative phosphorylation component of the electron transport chain.

The conditions that allow the study of the activity of a component of the mitochondrial electron transport chain or of any associated protein thereof depend on specific features of the mitochondrial preparation such as the type of specie, cellular origin or number of mitochondria among others. Therefore, the specific conditions for each type of sample must be optimized depending on the previously mentioned considerations. In a particular embodiment, an incubation buffer such as tris buffer (0.01-10.000 mM), phosphate buffer (0.01-10.000 mM), citrate buffer (0.01-10.000 mM) or bicarbonate buffer (0.01-10.000 mM) with (BSA (0.01-10 mg/ml), EDTA (0.01-10.000 mM), MgCl₂ (0.1-100 mM), pH= 4-9 and the redox probe such as DAB (0.01-100 mM) is used in the absence or in the presence of electron donors (0.01 mM-100 mM) with and without the tested drugs. The reaction is started by adding membranes homogenates (0.001-10 mg/ml) to the assay buffer (10-70°C) and the redox probe oxidation or reduction is measured at different times spectrophotometrically depending on the redox probe (DAB: 5-10 min at 400-595 nm) in a microtiter plate reader up to 960 min. In order to perform the assay using cell membrane microarrays or tissue sections, a scanner or any other device is used to digitalize the samples. Moreover, a specific device for colorimetric, fluorometric or any other type of labeling must be used depending on the redox probe.

In a particular embodiment, the first method of the invention further comprises a step (ib) after step (i) which comprises washing the sample comprising mitochondrial membranes to eliminate the electron donor and the redox probe, if said sample is a cell membrane array or a tissue sample. Said washing step allows eliminating the electron donor compound.

In a second step, the method comprises quantifying the signal obtained due to the oxidation or reduction of said redox probe.

The method of the invention also includes the quantification of the signal obtained due to the change in the redox state of the molecule owing to function of the mitochondrial respiratory chain. The signal can be quantified by means of any technique described in the current state of the art and well known by persons skilled in the art, such as colorimetric, fluorometric, photometric, electroanalytic or radiometric techniques, depending on the redox probe used.

In a preferred embodiment, the quantification is performed by photometric techniques. A skilled person in the art knows the conditions for identifying and measuring the presence of oxidation of the redox probe.

As a way of illustrative non-limitative examples, if the redox probe is DAB, its oxidation may be detected by measuring spectrophotometrically at 460 or any other range of absorbance suitable for this compound. The score given is a numerical representation of the intensity of the staining of the sample, and represents the amount of oxidation of the redox probe.

According to the first method of the invention oxidation of the redox probe is quantified if an electron donor probe is used as the redox probe. In said case the activity of a component of the electron transport chain or any associated protein thereof is stimulated if there is a decrease in the oxidation of said redox probe, and the activity of a component of the electron transport chain or any associated protein thereof is inhibited if there is an increase in the oxidation of said redox probe. A skilled person in the art can understand that the decrease in the oxidation of said redox probe is produced as long as there is an electron donor compound of a component of the electron transport chain or any associated protein thereof, which promotes an electron flux through the electron transport chain that reduces the oxidized components required by the redox probe.

Alternatively, the reduction of the redox probe is quantified if an electron acceptor probe is used as the redox probe. In said case the activity of a component of the electron transport chain or any associated protein thereof is stimulated if there is an increase in the reduction of said redox probe, and the activity of a component of the electron transport chain or any associated protein thereof is inhibited if there is a decrease in the reduction of said redox probe.

"Decrease" as used herein, relates to a reduction of a value more than at least 5 % 6%, 7%, 8%, 9%, 10%, 15%, 20%, 25%, 30%, 35%, 40%, 45%, 50%, 55%, 60%, 65%, 70%, 75%, 80%, 85%, 20 90%, 95%, 99%, or 100% compared to the value obtained in the absence of the condition or compound to be tested.

"Increased", as used herein, relates to an enhance of a value more than at least 5 % 6%, 7%, 8%, 9%, 10%, 15%, 20%, 25%, 30%, 35%, 40%, 45%, 50%, 55%, 60%, 65%, 70%, 75%, 80%, 85%, 20 90%, 95%, 99%, or 100% compared to the value obtained in the absence of the condition or compound to be tested.

### Method for determining the effect of a compound on the activity of a component of the mitochondrial ETC or any associated protein thereof.

In a second aspect, the invention relates to a method for determining the effect of a compound on the activity of a component of the electron transport chain or any associated protein thereof (second method of the invention), comprising:
(i) putting into contact the compound with a sample comprising membranes containing a component of the electron transport chain or any associated protein thereof in the presence of a redox probe, and at least one electron donor compound of any of said component or protein, in conditions allowing the interaction between said compound and a component of the electron transport chain or any associated protein thereof present in said sample, between said electron donor compound and its corresponding component of the electron transport chain or any associated protein thereof and between said redox probe and a oxidative phosphorylation component of the electron transport chain; and
(ii) quantifying the signal obtained due to the oxidation or reduction of said redox probe, wherein if the activity of a component of the electron transport chain or any associated protein thereof is increased, the compound is an inductor of one of the component of the electron transport chain or any associated protein thereof, and if the activity of a component of the electron transport chain or any associated protein thereof is reduced, the compound is an inhibitor of a component of the electron transport chain or any associated protein thereof.

"Method for determining the effect of a compound" as used herein, relates to a method for determining whether a compound is capable of inhibiting the activity of a component of mitochondrial ETC or any associated protein thereof or is capable of enhancing said activity. Inhibiting the activity, as used herein relates to a reduction in the activity by at least 5 percent, 10 percent, 15 percent, 20 percent, 25 percent, 30 percent, 35 percent, 40 percent, 45 percent, 50 percent, 60 percent, 70 percent, 80 percent, 85 percent, 90 percent, or at least 95 percent compared to the activity in the absence of the compound to be tested, or compared to a reference value. Alternatively, enhancement of the activity, as used herein relates to an increase in the activity by at least 5 percent, 10 percent, 15 percent, 20 percent, 25 percent, 30 percent, 35 percent, 40 percent, 45 percent, 50 percent, 60 percent, 70 percent, 80 percent, 85 percent, 90 percent, or at least 95 percent compared to the activity in the absence of the compound to be tested or compared to a reference value.

In a first step, the method for determining the effect of a compound on the activity of a component of the mitochondrial ETC or any associated protein thereof comprises putting into contact the compound with a sample comprising membranes containing a component of the electron transport chain or any associated protein thereof in the presence of a redox probe and at least one electron donor compound of any of said component or protein.

Compound to be tested according to the present invention may be a chemical compound or a biological product obtained by chemical synthesis and/or isolated from a live or dead organism, and therefore includes native compounds or synthetic compounds, and derivatives thereof. Examples of candidate compounds include drugs, molecules with affinity and selectivity for the previously described components involved in the mitochondrial oxidative phosphorylation process or any of the proteins that modulate its function, including antisense antibodies or fragments thereof.

In a preferred embodiment, the second method of the invention is for determining the effect of a compound on the activity of mitochondrial supercomplex.

Step (i) and (ii) may be performed as previously described in relation to the first method of the invention and all the particular embodiments apply to the method for determining the effect of a compound.

In a preferred embodiment, samples suitable in the present invention are membrane or cell solutions, microarrays, tissue sections or isolated mitochondria. In a preferred embodiment, the sample comprises a tissue sample, whole cells, organelles or isolated mitochondrial or isolated mitochondrial membranes. In another preferred embodiment, the sample is a cell membrane microarray. In another preferred embodiment, the sample is a mitochondrial membrane preparation (MMP). In a more preferred embodiment, said MMP is isolated from heart. In another preferred embodiment, the sample is a tissue sample, whole eukaryotic or prokaryotic cells, organelles, cellular membranes, liposomes, or isolated mitochondrial membranes or is a cell membrane microarray.

In a particular embodiment, the second method of the invention further comprises step (Ib) after step (I) which comprises washing the sample comprising mitochondrial membranes to eliminate the electron donor and the redox probe, if said sample is a cell membrane array or a tissue sample.

In a preferred embodiment, the redox probe is an electron donor probe. In another preferred embodiment the electron donor is a chromogenic redox probe. In an even more preferred embodiment, the electron donor probe is selected from the group consisting of 3,3'-diaminobenzidine (DAB); N,N,N',N'-Tetramethyl-p-phenylenediamine dihydrochloride (TMPD), ascorbic acid, 2,2'-azino-bis(3-ethylbenzothiazoline-6-sulphonic acid) (ABTS), o-phenylenediamine dihydrochloride (OPD), 4-aminoantipyrine, hyidroquinone (HQ), 3,3',5,5'-Tetramethylbenzidine (TMB), Amplex Red, 3-amino-9-ethylcarbazole (AEC), homovanillic acid, pyrogallol, p-aminophenol (PAP), N,N-diethyl-p-phenylenediamine sulfate (DPD) or 1,2,3-trihydroxybenzene (THB), among others and any derivative thereof. In a more preferred embodiment, the redox probe is 3,3'-diaminobenzidine (DAB).

In another preferred embodiment, the redox probe is an electron acceptor probe. In a more preferred embodiment, the electron acceptor probe is selected form the group consisting of 2,6-dichloroindophenol (DCIP); 2,6-Dichlorophenolindophenol (DCPIP); tetrazolium salts such as nitroblue tetrazolium (NBT) and any derivative thereof.

In another particular embodiment, of the method for determining the effect of a compound on the activity of a component of the electron transport chain or any associated protein thereof the electron donor compound of a component of the electron transport chain or protein associated thereof is a substrate of a component of the electron transport chain or protein associated thereof. In another preferred embodiment the substrate of complex I is NADH or NADPH, a substrate of complex II is succinate or FADH2, substrates of complex III are quinol, ferri cytochrome c or duroquinol, substrates of complex IV are TMPD or ascorbate, a substrate of complex V is ADP, a substrate of fatty acid β-oxidation enzymes are palmitoyl-CoA or any other acyl-CoA, a substrate of glycerol 3-phosphate dehydrogenase is glycerol 3-phosphate and a substrate of dihydroorotate dehydrogenase is dihydroorotate.

In a preferred embodiment, substrates of at least 2 complexes are used in the present invention. Particularly a substrate of complex I and a substrate of complex II; a substrate of complex I and a substrate of complex III; a substrate of complex I and a substrate of complex IV; a substrate of complex I and a substrate of complex V; a substrate of complex II and a substrate of complex III; a substrate of complex II and a substrate of complex IV; a substrate of complex II and a substrate of complex V; a substrate of complex III and a substrate of complex IV; a substrate of complex III and a substrate of complex V; or a substrate of complex IV and a substrate of complex V.

In another preferred embodiment, substrates of at least 3 complexes are used, particularly a substrate of complex I, a substrate of complex II and a substrate of complex III; a substrate of complex I, a substrate of complex II and a substrate of complex IV; a substrate of complex I, a substrate of complex II and a substrate of complex V; a substrate of complex I, a substrate of complex III and a substrate of complex IV; a substrate of complex I, a substrate of complex III and a substrate of complex V; a substrate of complex I, a substrate of complex IV and a substrate of complex V; a substrate of complex II, a substrate of complex III and a substrate of complex IV; a substrate of complex II, a substrate of complex III and a substrate of complex V; a substrate of complex II, a substrate of complex IV and a substrate of complex V; or a substrate of complex III, a substrate of complex IV and a substrate of complex V.

In another preferred embodiment, substrates of at least 4 complexes are used, particularly a substrate of complex I, a substrate of complex II, a substrate of complex III and a substrate of complex IV; a substrate of complex I, a substrate of complex II, a substrate of complex III and a substrate of complex V; a substrate of complex I, a substrate of complex II, a substrate of complex IV and a substrate of complex V; a substrate of complex I, a substrate of complex III, a substrate of complex IV and a substrate of complex V; or a substrate of complex II, a substrate of complex III, a substrate of complex IV and a substrate of complex V.

In another preferred embodiment substrates of at least 5 complexes are used, particularly a substrate of complex I, substrate of complex II, substrate of complex III, substrate of complex IV and a substrate of complex V.

In another preferred embodiment substrates of at least 5 complexes and of the associated enzymes of the ETC are used, particularly a substrate of complex I, substrate of complex II, substrate of complex III, substrate of complex IV, a substrate of complex V, a substrate of fatty acid β-oxidation enzymes, a substrate of glycerol 3-phosphate dehydrogenase and a substrate of dihydroorotate dehydrogenase.

In a more preferred embodiment, the method comprises a substrate of complex I and a substrate of complex II. In an even more preferred embodiment, the substrate of complex I is NADH or NADPH and the substrate of complex II is succinate or FADH2.

Step (ii) comprises quantifying the signal obtained due to the oxidation or reduction of said redox probe. In a particular embodiment, step (ii) is performed by a colorimetric, fluorometric, photometric, electroanalytic or radiometric method.

In addition, the method for determining the effect of a compound on the activity of a component of the ETC or any associated protein thereof may comprise an additional step of comparing the signal obtained in step (ii) with a reference value, in order to determine if the compound is capable of increasing or reducing the activity of said component or associated protein thereof.

"Reference value", as used herein relates to a laboratory value used as a reference for the values/data obtained from samples. The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample, such as, for example, a value obtained from a sample of study but obtained at a previous point in time. The reference value can be based on a high number of samples, such as the values obtained in a population of samples or based on a pool of samples including or excluding the sample to be tested. In a yet more preferred embodiment, if the activity of a component of the mitochondrial ETC or associated protein thereof is studied in isolated mitochondrial membranes, then the reference value is obtained from mitochondrial membranes. In a particular embodiment, the reference value of the signal obtained due to the oxidation of said redox probe in a mitochondrial functional assay according to the invention in the absence of the tested compound.

According to the method for determining the effect of a compound on the activity of a component of the electron transport chain or any associated protein thereof, if the activity of a component of the electron transport chain or any associated protein thereof is increased, the compound is an inductor of one of the component of the electron transport chain or any associated protein thereof, and if the activity of a component of the electron transport chain or any associated protein thereof is reduced, the compound is an inhibitor of a component of the electron transport chain or any associated protein thereof,

According to the method for determining the effect of a compound on the activity of a component of the electron transport chain or any associated protein thereof if the redox probe is an electron donor probe, the activity of a component of the electron transport chain or any associated protein thereof is stimulated if there is a decrease in the oxidation of said redox probe, and the activity of component of the electron transport chain or any associated protein thereof is inhibited if there is an increase in the oxidation of said redox probe. A skilled person in the art can understand that the activation evoked by a compound is triggered by the facilitation of the electron flux through the ETC and associated proteins thereof generated by a substrate or other electron donor of any of the mitochondrial components, while the inhibition is mediated by interfering said electron flux.

Alternatively, when the component of the electron transport chain is a supercomplex, the activity is considered inhibited by a compound if the time period which the redox probe oxidation is inhibited by a substrate or other electron donor of any of the mitochondrial components that integrate the supercomplex structure, is increased compared to the redox probe oxidation in the absence of said compound. A skilled person in the art can understand that said increase in the time period triggered by the compound is due to the decrease in the oxidation of the substrate by the mitochondrial component of said supercomplex, which remains associated in the supercomplex structure, hiding the supercomplex components required for the oxidation of the redox probe, until the substrate is totally consumed or the activity of said supercomplex component is stopped.

Alternatively if the redox probe is an electron acceptor probe, the activity of a component of the electron transport chain or any associated protein thereof is stimulated if there is an increase in the reduction of said redox probe, and the activity of a component of the electron transport chain or any associated protein thereof is inhibited if there is a decrease in the reduction of said redox probe.

The methods described herein are useful for identifying compounds that can be used to treat certain diseases and alter mitochondrial states. Compounds that increase mitochondrial respiration or other mitochondrial output can be identified by the present methods by testing compounds for increasing the activity of a component of the electron transport chain or any associated protein thereof. Such compounds can be used to increase the overall fitness of an animal or improve the condition of a diseased tissue. In addition, compounds that decrease the energy output of the mitochondria and therefore decrease the energy available to cells can be identified by the methods of the present invention. Compounds that decrease mitochondrial respiration or other mitochondrial output are useful to decrease the division and spread of cancer cells.

All the terms and embodiments previously described are equally applicable to this aspect of the invention.

### Kit of the invention

In another aspect, the invention relates to a kit comprising a redox probe, at least one electron donor compound of a component of the electron transport chain or of a protein associated thereof and optionally comprising a sample comprising mitochondrial membranes.

In the context of the present invention, "kit" is understood as a product containing the different reagents necessary for carrying out the methods of the invention packed so as to allow their transport and storage. Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instruction.

In a preferred embodiment, a redox probe and at least o electron donor compound of component of the electron transport chain or protein associated thereof comprise at least 10%, at least 20%, at least 30%, at least 40%, at least 50%, at least 60%, at least 70%, at least 80%, at least 90% or at least 100% of the total amount of reagents forming the kit.

In a preferred embodiment, the redox probe is an electron donor probe, more preferably is 3,3'-diaminobenzidine. In another preferred embodiment, the redox probe is an electron acceptor probe.

In another preferred embodiment, the electron donor compound of component of the electron transport chain or protein associated thereof is a substrate of component of the electron transport chain or protein associated thereof, more preferably is selected from the group consisting of a substrate of complex I, a substrate of a complex II, a substrate of complex III, a substrate of complex IV, a substrate of complex V a substrate of fatty acid β-oxidation enzymes, a substrate of glycerol 3-phosphate dehydrogenase and a substrate dihydroorotate dehydrogenase.

In another preferred embodiment, the kit comprises a substrate of complex I and and/or a substrate of a complex II. In a more preferred embodiment, the kit comprises NADH as the substrate as complex I and succinate as the substrate of complex II.

In another preferred embodiment, the kit of the invention further comprises a sample comprising mitochondrial membranes. Alternatively, the kits of the invention may comprise cells and medium for use in the assay in an immediately usable or readily reconstituted form and preferably any other reagents necessary to ensure the activity of a component of the mitochondrial ETC or any associated protein thereof. Optionally, the kit includes a known mitochondrial oxidizing or reducing agent. Also optionally, the kit further contains a detection device to facilitate determination of whether the candidate compound is a modulating compound. Also, the kit optionally contains multi-well plates or test tubes for running the assay. The kit may include a vial or vessel containing cells and an ampule or vial containing growth medium to sustain the cells. Such a kit may also include photographic film or other detection device

The kit of the invention may further comprise an assay buffer, preferably a tris buffer (0.01-10.000 mM), phosphate buffer (0.01-10.000 mM), citrate buffer (0.01-10.000 mM) or bicarbonate buffer, among others

In another aspect, the invention relates to the use of a kit of the invention in a method for determining the activity of a component of the mitochondrial ETC or any associated protein thereof according to the invention or in a method for determining the effect of a compound on the activity of component of the mitochondrial ETC or any associated protein thereof according to the invention.

All the terms and embodiments previously described are equally applicable to this aspect of the invention.

The invention will be described by way of the following examples which are to be considered as merely illustrative and not limitative of the scope of the invention.

### Examples

### Materials and methods

### Rats

Male Sprague-Dawley rats (250-300 g) were purchased from Harlan (Barcelona, Spain) and maintained under standard conditions (food and water ad libitum; 12h/12h light/dark cycle, light on 7 a.m.) according to the institutional guidelines and Directive 86/609/EEC. After being deeply anesthetized with a ketamine/xylazine cocktail (60 mg/kg of Ketamine-HCl and 5 mg/kg Xylazine-HCl i.p.), rats were decapitated and the heart of each animal was quickly removed at 4°C to avoid protein denaturation. Hearts were stored at -80 °C.

### Drugs

DAB, NBT, DCIP, NADH, NADPH, NAD, cytochrome c, decylubiquinone, rotenone, antimycin, myxothiazol, CCCP, DNP, digitonin, deoxycholate, SDS, oxalacetate, DAB, NBT, DCIP and other chemicals used in this study were purchased from Sigma Aldrich (St-Louis, IL, USA).

DAB, NBT, DCIP, NADH, NADPH, NAD, cytochrome c, digitonin, deoxycholate and SDS were dissolved in water. Decylubiquinone, rotenone, antimycin, myxothiazol, CCCP and DNP were dissolved in DMSO. Corresponding vehicle solutions were used in control experiments.

### Isolation of freshly mitochondria

The hearts of rat littermates were dissected and mitochondria were purified using Kristal and coworkers protocol. Briefly, hearts were extracted in ice-cold isolation buffer (250 mM sucrose, 50 mM Tris-HCl, 3 mM MgCl2 1 mM EGTA, pH 7.4) and homogenized with a Teflon-glass grinder. The crude homogenates were centrifuged at 500 x g for 5 min (4°C, Microfuge® 22R centrifuge, Beckman Coulter). The supernatant was then centrifuged at 14,000 x g (4°C). The pellet was washed in 20 volumes of isolation buffer and re-centrifuged under the same conditions. The experiments using freshly isolated heart mitochondria were performed within 3 h following purification.

### Isolation of mitochondrial membranes

Hearts were homogenized using a Teflon-glass grinder (Heidolph RZR 2020) in 20 volumes of homogenized buffer (1 mM EGTA, 3 mM MgCl2, and 50 mM Tris-HCl, pH 7.4) supplemented with 250 mM sucrose. The crude homogenate was subjected to a 1000x g for 8 min, and the resultant supernatant was centrifuged again at 18,000x g for 15 min (4°C, Microfuge® 22R centrifuge, Beckman Coulter). The pellet was washed in 20 volumes of homogenized buffer using an Ultra-Turrax® (T10 basic, IKA) and re-centrifuged under the same conditions. The homogenate aliquots were stored at -80 °C until they were used. Protein concentration was measured by the Bradford method and adjusted to the required concentrations.

### Mitochondrial malate dehydrogenase. activity

The standard assay medium for measurement of malate dehydrogenase activity consisted of 20 mM MOPS, 0.3 mM oxalacetate and 0.1 mM NADH, pH 7,5 (25°C). The reaction was started by the addition of 0.1 mg/ml of freshly isolated mitochondria or membrane homogenates. The change in absorbance was measured at 340 nm on Scan it for Multiscan spectrophotometer (Thermo Scientific). Control experiments were performed to exclude any interference or substrate interaction.

NADH-ubiquinone oxidoreductase (complex I) and succinate dehydrogenase (complex II) activity assay.

CI and CII assays were performed based on a previous protocol with some modifications. The reaction mixture consisted of potassium phosphate buffer (0.8M K2HPO4, 0.2M KH2PO4, pH 7.4), 3 mg/ml BSA, 160 µM 2,6-dichloroindophenol (DCIP) (Sigma) together with the CI or CII substrates. The reaction was started by the addition of 0.1 mg/ml membranes homogenates (25°C) in the absence or in the presence of decylubiquinone and ETC inhibitors. DCIP reduction was measured every 30 seg spectrophotometrically at 595 nm in a Multiskan microtiter plate reader (Thermo Scientific). Control experiments were performed to exclude any interference or substrate interaction.

### Superoxide production

The assay medium for measurement of superoxide production consisted of a 50 mM phosphate buffer (Na2HPO4, NaH2PO4, pH 7,4), 0,5 mg/ml nitro blue tetrazolium (NBT) as a redox dye and CI or CII substrates. The reaction was started by the addition of 0.1 mg/ml membranes homogenates for 360 min (25°C) with or without decylubiquinone and ETC inhibitors. The NBT reduction evoked by superoxide was measured every 10 min spectrophotometrically at 595 nm in a Multiskan microtiter plate reader (Thermo Scientific).

### Cell Membrane Microarray development

Membrane homogenates were resuspended in buffer and they were printed (4 nL per spot, 3-5 replicates per sample) together with internal patrons onto glass slides using a non-contact microarrayer (Nano_plotter NP 2.1). Microarrays were stored at -20°C until they were used.

### Activity assay of the of individual complexes, supercomplexes and associated proteins of the mitochondrial ETC

The assay buffer f consisted of a phosphate buffer (pH 7,4), EDTA, Cl₂Mg, BSA and 3,3'-diaminobenzidine (DAB) in the absence or in the presence of CI and CII substrates and the tested drugs. The reaction was started by adding 0.1 mg/ml membranes homogenates to the assay buffer (25°C) or putting into contact cell membrane microarrays with assay buffer. DAB oxidation was measured every 5-10 min spectrophotometrically at 460 and 595 nm in a Multiskan microtiter plate reader (Thermo Scientific) for 960 min in the case of homogenates

### Image processing

A Leica DM500 microscope and Leica ICC50 HD camera were coupled to the capturing software LAS EZ 2.0.0. Microphotographs were captured with the same light parameters and the final image was assembled with the software Adobe Photoshop CS5 (Adobe Systems Incorporated, California, USA). Microarrays were also scanned using a scanner (Epson Perfection V750 Pro) and quantified with the MAPIX software (version 7.3.1).

### Statistical analyses

Data handling and analysis was carried out using Excel and GraphPad software (version 6.0). Results were expressed as means of independent data points ± S.E.M. In sake of clarity, biochemical data (Complex I and II activities, superoxide production and supercomplex activity) are presented as percentage of controls with or without the application of ETC inhibitors. Data were analyzed using paired Student's t-test, one-way (followed by Tukey's post hoc test) or two-way ANOVA (followed by Bonferroni's post hoc test), as appropriate.

### EXAMPLE 1- Study of the pharmacological effect of different inhibitors of the electron transport chain on the activity of mitochondrial supercomplexes

A set of specific inhibitors of the electron transport chain (rotenone, myxothiazol and antimycin A) has been studied for the purpose of determining the specific activity of each molecule on supercomplex activity. In addition the effects on supercomplex activity of the specific inhibitor of the complex III antimycin A has been studied in microarrays consisted of membrane homogenates isolated from rat heart cells.

The inventors present a methodological approach that allows studying SC function in mitochondrial membrane preparations (MMP) isolated from rat heart, based on the study of the electron donor 3,3'-diaminobenzidine (DAB) in presence of the CI and CII substrates, NADH and succinate. The electron input generated by these substrates competes with that produced by DAB for cyt c, revealing that ETC keeps functional for several hours in MMP as well as in cell membrane microarrays (Fig. 1a, b and Fig. 6a, b). Furthermore, the full blockage observed with NADH, in comparison with the partial inhibition obtained with succinate, indicates that the electron flux efficiency is higher when electrons are provided by CI, supporting the substrate channelling in SC. In this context, dUQ induces a slight reduction of the NADH evoked inhibition of DAB oxidation compared to that produced in the presence of 1 mM succinate, according to the lower diffusion distance expected for electron carriers in SC (Fig. 1c, d). At higher concentrations of succinate, in which its concentration is not a limiting factor, dUQ potentiates the DAB oxidation blockage until NADH does, in accordance with the diffusion model proposed for complexes which are not associated in SC (Fig. 1e)

The addition of exogenous soluble cyt c raises DAB oxidation in a dose-dependent manner (Fig. 1f). However, exogenous cyt c cannot bypass the full inhibition caused by NADH on DAB oxidation even under saturation conditions (Fig. 1g). Once NADH is depleted, DAB can donate again its electrons to the cyt c reaching the maximal effect observed in the absence of the CI substrate (Fig. 1g). Therefore, the preferential electron flux observed across the SC, even when soluble cyt c is present, supports the substrate channelling between complexes III and IV via cyt c. Moreover, the full inhibition of DAB oxidation induced by NADH points out that the whole population of CIII and CIV is coupled to CI while CI is active, suggesting that SC are dynamics structures whose coupling depends on the substrate availability, in the same way as oxygen availability modifies the composition of mitochondrial SC in plants.

The exogenous cyt c also reduces the effect promoted by CI and CII substrates on DAB oxidation (Fig. 1g, h). In particular, exogenous cyt c decreases the time period in which a specific NADH concentration induces the DAB oxidation blockage, indicating that the presence of this electron carrier accelerates the NADH consumption rate displayed by CI (Fig. 1i). Therefore, these data show that soluble cyt c can diffuse into SC structure even when it is active, remaining there at least until its reoxidation. In agreement with this assumption, X-ray structures and cryo-EM images of bovine SC reveal that the binding sites for cyt c between CIII and CIV are open to the membrane allowing its diffusion. Moreover, functional studies on Saccharomyces cerevisiae indicate that soluble cyt c can be bound to the SC enabling a direct electron transfer via the attached cyt c which is faster than that produced by the electron equilibration with the cyt c pool.

Indeed, cyt c can be associated with acidic phospholipids such as cardiolipins, which play a very important role in mitochondrial complex and SC activity. However the physiological relevance of the endogenous attached cyt c has not been well characterized owing to requirement of detergents in the isolation steps that might promote its release. In this sense, digitonin induces a biphasic action on basal DAB oxidation as well as an enhancement on the NADH inhibition period (Fig. 2a, b), effects also observed with SDS, Tween20 and sodium deoxycholate. The exogenous addition of cyt c mitigates these effects (Fig. 2c, d), suggesting that detergents modify both the availability and the content of cyt c, prior affecting the individual complex activity such as CIV7 and supercomplex function.

Some specific inhibitors of ETC evoked similar effects on cyt c. In this context, antimycin A induces the reduction in the maximal effect of DAB oxidation (Fig. 2e), as well as the enhancement of the NADH-inhibition period (Fig. 2f). Furthermore, the addition of soluble cyt c mitigates both (Fig. 2g, h), indicating that antimycin A promotes the release of the endogenous attached cyt c. Antimycin A is a specific inhibitor of CIII QN site that induces cell death by blocking ETC, with the subsequent growth in ROS formation and cyt c release. In accordance with these actions, antimycin A blocks the electron flux generated by CII to cyt c, avoiding, in this way, the decrease in DAB oxidation observed in the presence of succinate (Fig. 2i). Myxothiazol, a Qp inhibitor, also promotes the blockage of the succinate-derived electron input with similar efficiency as antimycin A does (Fig. 2i).

However, in presence of NADH, these inhibitors of CIII, as well as rotenone enhances the substrate evoked inhibition of DAB oxidation (Fig. 2h, 3a-b), pointing to a reduction in the NADH consumption rate. Thus, these data reveal that the coupling among the mitochondrial complexes which integrate SC is maintained as long as CI is active and in spite of blockade of the electron flux along the ETC. In this context, the shift in the reaction equilibrium to the active conformation, using soluble cyt c as electron carrier, accelerates the NADH consumption rate even in the presence of these inhibitors (Fig. 3c). Moreover, the electron carrier dUQ shows a biphasic effect, reducing the inhibitor mediated effects at 10 µM whilst potentiating those evoked by rotenone and antimycin A at 50 µM (Fig. 3d-f). Thus, dUQ might compete with the inhibitor for the binding site at low concentrations whereas at higher concentrations, the oxidized form of dUQ might couple to the complexes stabilizing a less active SC conformation.

In order to clarify this action, the enzymatic activities of CI and CII were evaluated using DCIP, a redox dye which withdraws electrons preferentially from ubiquinol. The inventors observed that myxothiazol but not antimycin A efficiently blocked DCIP reduction in the presence of NADH (Fig. 3g), whilst antimycin A but not myxothiazol did it when the substrate was succinate (Fig. 3h). This dependence on the substrate of the CIII inhibitors competing effects might suggest that CI mainly uses a specific CoQ pool located in the outer site of the internal mitochondrial membrane, while CII uses a different pool, located in the inner site. In this sense, the addition of dUQ blocks the effect of antimycin A (Fig. 3i) but it does not produce the same effect with myxothiazol (Fig. 3j) suggesting that the diffusion of dUQ to the SC is limited as long as CI and CIII are coupled, in contrast to what happens with CII, which does not form SC. However, the superoxide production induced by these inhibitors of CIII is quite similar, using NADH (Fig. 3k) or succinate (Fig. 3l), indicating that once they are bound, the source of ubiquinol does not have any effect in the process. Moreover, the superoxide production triggered by antimycin A presents a biphasic effect in which a maximum was observed in low micromolar range, while higher concentrations reduced it, probably due to the fully inhibition of the CIII by the binding of antimycin A to its second binding site. Rotenone induces superoxide formation when the substrate employed is NADH with similar efficiency than myxothiazol and antimycin A (Fig. 3k). This superoxide production is increased in the presence of dUQ in agreement to the thesis that semiquinone intermediates are important for ROS generation. Moreover, rotenone does not block the enhancement in the speed of ROS production provoked by dUQ, indicating that both molecules can bind to CI at the same time.

To better understand the influence of CI state on ROS formation and SC function without using exogenous electron carriers and inhibitors that share their binding site, the inventors used NADPH as CI substrate. This nucleotide is a cofactor of the 39 kDa subunit of CI which has been proposed to act as redox sensor. Moreover, NADPH increases [³H]-dihydrorotenone binding sites at the same level as NADH does but its action as a substrate of CI is still under discussion. In this context, the inventors observed that NADPH inhibits DAB oxidation in a similar way as that triggered by NADH in the presence of inhibitors such as rotenone (Fig. 4a), denoting that CI's consumption rate of both nucleotides is very similar (Fig. 4b). In fact, exogenous cyt c increases the NADPH consumption rate while NAD reduces it (Fig. 4c, d). Moreover, the NADPH-inhibition period is enhanced by dUQ in a similar way as what happens with rotenone and antimicyn A at high concentrations of dUQ (Fig. 3d-e, 4e). Thus, the activation of CI by NADPH induces the coupling of SC without electron donation to ETC probably because electrons are redirected to ROS production (Fig. 4f).

As the substrate oxidation involves not only the electron transfer but also the proton translocation, the inventors decided to use Zinc to block CI proton channels, and the protonophores, DNP and CCCP to discard the influence of the proton gradient. On one hand, DNP does not alter DAB oxidation either in basal condition or in the presence of NADH (Fig. 5a, b), confirming that the uncoupling of the proton gradient does not affect the SC function in our "in vitro" assay which lacks of whole mitochondrias. By contrast, CCCP induces changes in both (Fig. 5c, d) promoting a less efficient electron flow along ETC at high doses, probably by the direct interaction with CIII or CIV. On the other hand, Zinc reduces basal DAB oxidation and enhances the inhibition period evoked by NADH (Fig. 5e, f). However, this effect is less potent than that produced by rotenone, according to the partial blocking of the ETC proposed for this ion (Fig. 5g). Therefore, these data support that once CI is in the active conformation, SC remains coupled as long as there is still substrate even when the electron transfer or the proton translocation pathway are blocked.

However, the SC functionality and the influence in their function of exogenous electron carriers and inhibitors depend on the specie. In this sense, dUQ significantly reduces the NADH-dependent inhibition period of DAB oxidation in MMP isolated from hearts of cynomolgus monkey (Fig. 5h), compared to that observed in rat (Fig. 1c). Likewise, the partial blockage of the rotenone effect induced by dUQ measured in rat was not detected in monkey at least at similar concentrations, indicating that the CoQ content or the accessibility of this electron carrier to the SC structure varies according to the specie (Fig. 3d, 5i).

In summary, these data reveal important functional aspects of the mitochondrial supercomplexes that support the relevance of these macrostructures, although further studies will be necessary to characterize them in different species.

## Claims

**1.** A method for determining the activity of a component of the electron transport chain or any associated protein thereof, which comprises:
(i) putting into contact a sample comprising membranes containing a component of the electron transport chain or any associated protein thereof with a redox probe and at least one electron donor compound of any of said component or protein in conditions allowing the interaction between said electron donor compound and said corresponding component of the electron transport chain or associated protein thereof present in said sample and between said redox probe and a oxidative phosphorylation component of the electron transport chain; and
(ii) quantifying the signal obtained due to the oxidation or reduction of said redox probe.

**2.** A method for determining the effect of a compound on the activity of a component of the electron transport chain or any associated protein thereof, comprising:
(i) putting into contact the compound with a sample comprising membranes containing a component of the electron transport chain or any associated protein thereof in the presence of a redox probe, and at least one electron donor compound of any of said component or protein, in conditions allowing the interaction between said compound and a component of the electron transport chain or any associated protein thereof present in said sample, between said electron donor compound and its corresponding component of the electron transport chain or any associated protein thereof and between said redox probe and a oxidative phosphorylation component of the electron transport chain; and
(ii) quantifying the signal obtained due to the oxidation or reduction of said redox probe, wherein if the activity of a component of the electron transport chain or any associated protein thereof is increased, the compound is an inductor of one of the component of the electron transport chain or any associated protein thereof, and if the activity of a component of the electron transport chain or any associated protein thereof is reduced, the compound is an inhibitor of a component of the electron transport chain or any associated protein thereof.

**3.** The method according to any of claims 1 or 2 further comprising a step (ib) after step (i) which comprises washing the sample comprising mitochondrial membranes to eliminate the electron donor and the redox probe, if said sample is a cell membrane array or a tissue sample.

**4.** The method according to any of claim 1 to 3, wherein step (ii) is performed by a colorimetric, fluorometric, photometric, electroanalytic or radiometric method.

**5.** The method according to any one of claims 1 to 4 wherein said redox probe is an electron donor probe, wherein the activity of a component of the electron transport chain or any associated protein thereof is stimulated if there is a decrease in the oxidation of said redox probe, and the activity of a component of the electron transport chain or any associated protein thereof is inhibited if there is an increase in the oxidation of said redox probe or a method according to any of claims 2 to 4 wherein said redox probe is an electron donor probe, the activity of a component of the electron transport chain being a supercomplex is inhibited by the compound if the time period which the redox probe oxidation is inhibited is increased compared to the redox probe oxidation in the absence of said compound.

**6.** The method according to claim 5, wherein the electron donor probe is selected from the group consisting of 3,3'-diaminobenzidine (DAB); N,N,N',N'-Tetramethyl-p-phenylenediamine dihydrochloride (TMPD), ascorbic acid, 2,2'-azino-bis(3-ethylbenzothiazoline-6-sulphonic acid) (ABTS), o-phenylenediamine dihydrochloride (OPD), 4-aminoantipyrine, hyidroquinone (HQ), 3,3',5,5'-Tetramethylbenzidine (TMB), Amplex Red, 3-amino-9-ethylcarbazole (AEC), homovanillic acid, pyrogallol, p-aminophenol (PAP), N,N-diethyl-p-phenylenediamine sulfate (DPD) or 1,2,3-trihydroxybenzene (THB) and any derivative thereof.

**7.** The method according to any of claims 1 to 4 wherein said redox probe is an electron acceptor probe, wherein the activity of a component of the electron transport chain or any associated protein thereof is stimulated if there is an increase in the reduction of said redox probe, and the activity of a component of the electron transport chain or any associated protein thereof is inhibited if there is a decrease in the reduction of said redox probe.

**8.** The method according to claim 7 wherein the electron acceptor probe is selected from the group consisting of 2,6-dichloroindophenol (DCIP); 2,6-Dichlorophenolindophenol (DCPIP); tetrazolium salts such as nitroblue tetrazolium (NBT) and any derivative thereof.

**9.** The method according to any of claims 1 to 8 wherein the sample comprises a tissue sample, whole eukaryotic or prokaryotic cells, organelles, cellular membranes, liposomes, isolated mitochondrial membranes or is a cell membrane microarray.

**10.** The method according to any of claims 1 to 9 wherein the component of the electron transport chain is selected from the group consisting of complex I, complex II, complex III, complex IV and complex V and/or the associated protein thereof is selected from the group consisting of fatty acid β-oxidation enzymes, glycerol 3-phosphate dehydrogenase, dihydroorotate dehydrogenase and protein kinase A.

**10.** The method according to any of claims 1 to 9 wherein the electron donor compound of a component of the electron transport chain or protein associated thereof is a substrate of a component of the electron transport chain or protein associated thereof.

**11.** The method according to claim 10, wherein the substrate of complex I is NADH or NADPH, a substrate of complex II is succinate or FADH2, substrates of complex III are quinol, ferri cytochrome c or duroquinol, substrates of complex IV are TMPD or ascorbate, a substrate of complex V is ADP, a substrate of fatty acid β-oxidation enzymes are palmitoyl-CoA or any other acyl-CoA, a substrate of glycerol 3-phosphate dehydrogenase is glycerol 3-phosphate and a substrate of dihydroorotate dehydrogenase is dihydroorotate.

**12.** A kit comprising a redox probe, at least one electron donor compound of a component of the electron transport chain or of a protein associated thereof and optionally comprising a sample comprising mitochondrial membranes.

**13.** The kit according to claim 12, wherein the redox probe is 3,3'-diaminobenzidine.

**14.** The kit according to any of claims 12 to 13, wherein the electron donor compound of a component of the electron transport chain or protein associated thereof is selected from the group consisting of a substrate of complex I, a substrate of a complex II, a substrate of complex III, a substrate of complex IV, a substrate of complex V a substrate of fatty acid β-oxidation enzymes, a substrate of glycerol 3-phosphate dehydrogenase and a substrate dihydroorotate dehydrogenase.

**15.** Use of the kit according to any of claims 12 to 14 in a method according to any of claims 1 to 11.
